# EUROPEAN PATENT APPLICATION

(11) **EP 1 950 535 A1**
(43) Date of publication of application: **30.07.2008**
(21) Application number: 07113406.8
(22) Date of filing: 30.07.2007
(51) Int. Cl.: G01F 1/684

(54) **Method and system for dynamic compensation of bi-directional flow sensor during respiratory therapy**

(30) Priority: 07.08.2006 US 500257
(71) Applicant: General Electric Company, Schenectady, NY 12345 (US)
(72) Inventor: Christensen, David Allan, Cambridge, WI 53523 (US)
(74) Representative: Bedford, Grant Richard

(57) **Abstract**

The method and system includes a flow sensor including two hot wire gas flow sensing elements (32, 62) connected to identical, but independent electronic circuitry (20), positioned in parallel, transverse to gas flow (64) direction, and separated by a post (60) that serves to shadow the downstream element (32, 62). The flow sensors (32, 62) are dynamically compensated during respiratory therapy by use of digital to analog conversion, which provides for more accurate identification of flow direction and more accurate gas flow in volume measurements. The method and system incorporates the flow sensors (32, 62) in the y-piece of a breathing circuit.

## Description

The invention relates generally to the field of clinical systems. More particularly, the invention relates to the field of life support solutions.

In various aspects, the present invention relates to gas flow sensors employing a heated resistance wire, commonly called hot wire anemometers. Numerous applications require measurement of the flow rate of a gas or mixture of gases. One such application is in medical apparatus, such as ventilators, for measuring the flow rate of respiratory breathing gases expired by the subject. Measuring the flow rate of expired breathing gas is particularly difficult due to the wide range of instantaneous gas flow rates found during expiration, variations in the composition of the exhaled breathing gases, the moisture and sputum exhaled in the breathing gases, and for other reasons.

Hot wire gas flow sensors, or anemometers, have found use as expiratory breathing gas flow sensors in ventilators and similar equipment. In the simplest form of such a flow sensor, a thin, resistive wire, usually of platinum, is positioned in an airway flow conduit through which the expiratory gases pass. The wire typically extends transverse to the gas flow direction through the conduit. The platinum wire resistor forms one arm of a Wheatstone bridge circuit. The other arms of the bridge circuit contain other resistors, one or more of which may be variable. A power supply is connected across one pair of terminals of the bridge circuit and an indicator device is connected across the other pair of terminals of the bridge circuit.

Energization of the bridge circuit passes current through the platinum resistor to increase its temperature and cause it to become a "hot wire." The resistance of a platinum wire resistor is proportional to its temperature. As the gas flows past the hot wire, the wire is cooled, altering the resistance of the resistor. The resulting resistance imbalance in the bridge circuit, as sensed in the indicator device, is an indication of the flow rate of the gas passing the hot wire resistor.

In another embodiment of such an anemometer, as the resistance of the wire resistor changes due to gas flow, the energization of the bridge is altered to keep the current through the wire resistor constant. The voltage drop across the resistor becomes an indication of the gas flow rate.

Or, as the resistance of the wire resistor changes responsive to gas flow, the current through the platinum wire resistor is adjusted to keep its temperature, and hence its resistance, constant. The resulting voltage drop across the resistor is the indicator of the flow rate of the gas.

While highly suited as a means for measuring gas flow rates, certain problems have heretofore attended the use of hot wire anemometers. One problem arises during replacement of the wire sensing resistor due to breakage or other reasons. In practical embodiments of hot wire anemometers, the resistance of the sensing resistor inevitably varies from resistor to resistor. Replacement of a sensing resistor of one resistance with a sensing resistance of a different resistance has heretofore made it necessary to perform a calibration of the gases flow rate sensor, usually under field service conditions. To carry out the calibration most accurately requires a gas source that provides gas of a given composition over a known range of flow rates. This requirement has rendered calibration of hot wire gas flow rate sensors when the sensing resistor is replaced as awkward, time consuming, and expensive.

According to various aspects of the present invention, a method and system include a flow sensor including two hot wire gas flow sensing elements connected to identical, but independent electronic circuitry, positioned in parallel, transverse to gas flow direction, and separated by a post that serves to shadow the downstream element. The flow sensors are dynamically compensated during respiratory therapy by use of digital to analog conversion, which provides for more accurate identification of flow direction and more accurate gas flow in volume measurements. The method and system incorporates the flow sensors in the y-piece of a breathing circuit.

One aspect of the present invention is a system for dynamically compensating a bidirectional flow sensor, the system comprises a first and second sensing circuit, each of the first and second sensing circuits including a sensing element, wherein the first and second sensing elements are configured in parallel to one another and transverse to a gas flow, a post element configured between the first and second sensing elements, wherein the post element is further configured to shield the first sensing element when the gas flow is in an inspiration direction, and is configured to shield the second sensing element when the gas flow is in an expiration direction, wherein when the gas flow is in a zero condition between the inspiration direction and the expiration direction, an inspiration signal and an expiration signal collected by the first and second sensing elements are compensated to equal an ideal flow value. The system further comprises a y-piece configured to house the first and second sensing elements and the post element, wherein the y-piece shields the first and second sensing elements and the post element from a base flow and a digital to analog converter (DAC) coupled with the first sensing circuit and the second sensing circuit wherein an output voltage of the DAC compensates the inspiration and expiration signals and the first and second sensing circuits are bridge circuits that include a plurality of bridge arms, further wherein any of the plurality of bridge arms is the sensing element and the ideal zero flow value = 200 mV.

Another aspect of the present invention is a method of dynamically compensating a bi-directional sensor, the method comprises configuring a pair of sensing elements transverse to a gas flow, wherein the pair of sensing elements are configured in parallel to one another, configuring a post element between the pair of sensing elements with respect to the gas flow, measuring an inspiration signal and an expiration signal with each of the pair of sensing elements, comparing the inspiration signal and the expiration signal to an ideal flow value when the gas flow is in a zero condition and adjusting the inspiration signal and the expiration signal to equal the ideal flow value when the gas flow is in a zero condition wherein each of the pair of sensing elements is coupled to each one of a pair of sensing circuits, the post element is further configured to shield a first one of the pair of sensing elements when the gas flow is in an inspiration direction, and is configured to shield a second one of the pair of sensing elements when the gas flow is in an expiration direction. The method further comprises configuring the pair of sensing elements and the post element in a y-piece wherein the γ-piece shields the pair of sensing elements and the post element from the a base flow, wherein an output voltage of a digital to analog converter (DAC) adjusts the inspiration and expiration signals, wherein the DAC is coupled with the pair of sensing circuits wherein the pair of sensing circuits are bridge circuits that may include a plurality of bridge arms, further wherein any of the plurality of bridge arms is a sensing element wherein the ideal zero flow value equals 200 mV.

Yet another aspect of the present invention is an apparatus for dynamically compensating a bi-directional flow sensor, the apparatus comprises a storage media for storing a computer application, a processing unit coupled to the storage media, a pair of sensing elements coupled to a pair of sensing circuits, wherein the pair of sensing elements are configured in parallel to one another and transverse to a gas flow, and a post element configured between the pair of sensing elements, wherein when the gas flow is in a zero condition, an inspiration signal and an expiration signal collected by the pair of sensing elements are compared to an ideal flow value by the computer application, and the computer application adjusts a digital to analog converter so as to compensate the inspiration signal and expiration signal to equal the ideal flow value.

Various aspects and embodiments of the present invention will now be described in connection with the accompanying drawings, in which:
Figure 1 illustrates a schematic diagram of Wheatstone bridge circuitry.
Figure 2 illustrates a schematic diagram of Wheatstone bridge circuitry with compensation through digital to analog conversion.
Figure 3 illustrates a graphical representation of sensor hot wire elements with shadowing post.
Figure 4 illustrates a graphical representation of inspiratory flow ADC counts, expiratory flow ADC, and net flow in liter/min.
Figure 5 illustrates a flow chart according to an embodiment of a method of the present invention.

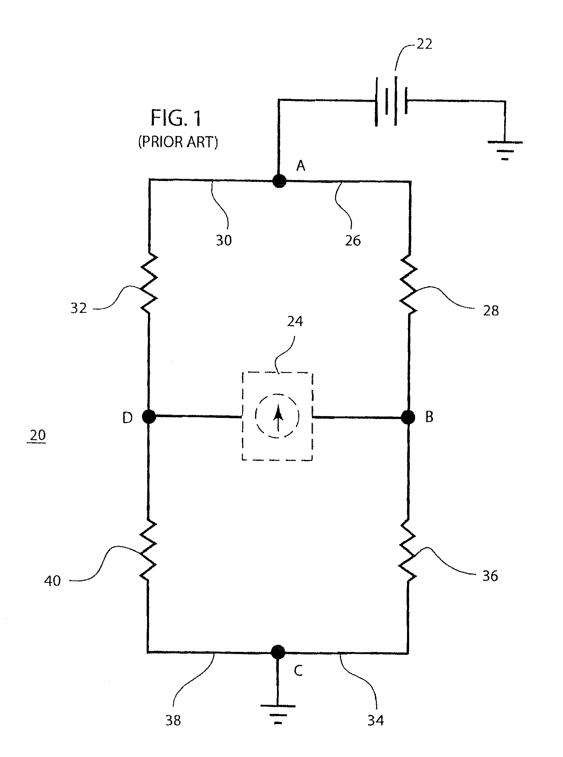

The construction and operating principles of a simple bridge circuit of the type employed in the sensor of an embodiment of the present invention are described below in connection with Fig. 1. The bridge circuit shown in Fig. 1 is commonly termed a Wheatstone bridge. Bridge circuit 20 has input terminals A and C connected to a voltage source 22 and a pair of output terminals B and D which may be connected to an output indicating device 24, such as a galvanometer. Bridge arm 26 extends between input terminal A and output terminal B and contains resistor 28. Bridge arm 30 extends between input terminal A and output terminal D and contains resistor 32. Bridge arm 34 extends between output terminal B and input terminal C and contains resistor 36. Bridge arm 38 extends between input terminal C and output terminal D and contains resistor 40.

The operation of bridge circuit 20 is as follows. Assume resistor 28 has a value of 100 ohms, resistor 36 has a value of 1,000 ohms, resistor 32 has a value of 300 ohms and resistor 40 has a value of 3,000 ohms. Voltage source 22 has a value of 1.5 v and will divide in the parallel current paths with 1.36 milliamps flowing in the path A, B, C and 0.45 milliamps flowing in the path A, D, C. The 1.36 milliamps current passing through the 1,000 ohm resistor 36 will provide a voltage drop of 1.36 volts across the resistor and at output terminal B. The 0.45 milliamp current through the 3,000 ohm resistor 40 will also provide a 1.36 volt potential drop across this resistor and at output terminal D. With the same voltage appearing at output terminal B and D, no current will flow through output device 24. Output device 24 will indicate that bridge circuit 20 is in the balance or null condition.

Assume, now, the resistance of resistor 40 changes to 2,000 ohms. The 1.5 volts of source 22 passing through the series connected resistor 32 and 40 of 300 ohms and 2,000 ohms, respectively, will provide a 0.65 milliamp current in the path A, D, C. The 0.65 milliamp current passing through the 2,000 ohm resistor 40 will provide a voltage drop of 1.3 volts across the resistor. The current and voltages existing in path A, B, C remain unchanged as does the 1.36 volt potential at output terminal B. The voltage at output terminal D is now 1.3 volts. There is thus a 0.06 volt (60 millivolt) potential difference existing at output terminals B and D, with output terminal B being positive with respect to output terminal D. This circumstance, when sensed by output device 24, indicates that bridge 20 is no longer in the balanced condition.

In an analogous manner the resistance of resistor 40 may increase to 4000 ohms. This causes a current of 0.35 milliamps to flow through path A, D, C. This 0.35 milliamp current when applied to resistor 40 of 4000 ohms, causes a voltage drop of 1.4 volts across the resistor and a corresponding voltage to appear at output terminal D. The voltage to appear at output terminal D. The voltage at output terminal B remains unchanged at 1.36 volts. There is now a 0.04 volt (40 millivolt) difference between the voltages at output terminal D being positive with respect to terminal B. This voltage difference, when applied to output device 24 also indicates an unbalanced condition of the bridge with the polarity of the voltage indicating the relative magnitude of the resistance of the resistor 40 with respect to the resistance of resistor 36.

The resistance of a resistor, such as one formed from platinum wire, changes with its temperature. Passing current through the resistor will heat the resistor. Exposing the resistor to a flowing gas will decrease the temperature and resistance of the resistor as the gas carries off heat. The change in temperature, and hence resistance of a resistor exposed to a gas flow is dependent on the mass flow rate (dm/dt) of the gas.

A bridge circuit in which one of the resistors, for example resistor 40, is a platinum wire exposed to the gas, may thus form a gas flow sensor. The platinum wire resistor is typically placed in a conduit or chamber for the gas and transverse to the direction of flow of the gas. In one embodiment of such a sensor, the amount of imbalance in the bridge circuit produced by the change of resistance of resistor 40 is an indication of the gas flow rate. Typically in such a gas sensor, the resistances of the resistor including wire resistor 40 would be considerably less than those described in explanatory fashion previously. For example, the resistance of resistor 40 would usually be about 3-5 ohms.

In another embodiment of a bridge circuit gas flow sensor, the output of the bridge can be applied to the input of a current regulating loop that maintains the bridge, and can be applied to the input of a current regulating loop that maintains the bridge in the constant current condition as the resistance of resistor 40 varies responsive to gas flow. The voltage across the variable resistance of the sensing resistor supplied with the constant current then becomes an indication of the gas flow rate.

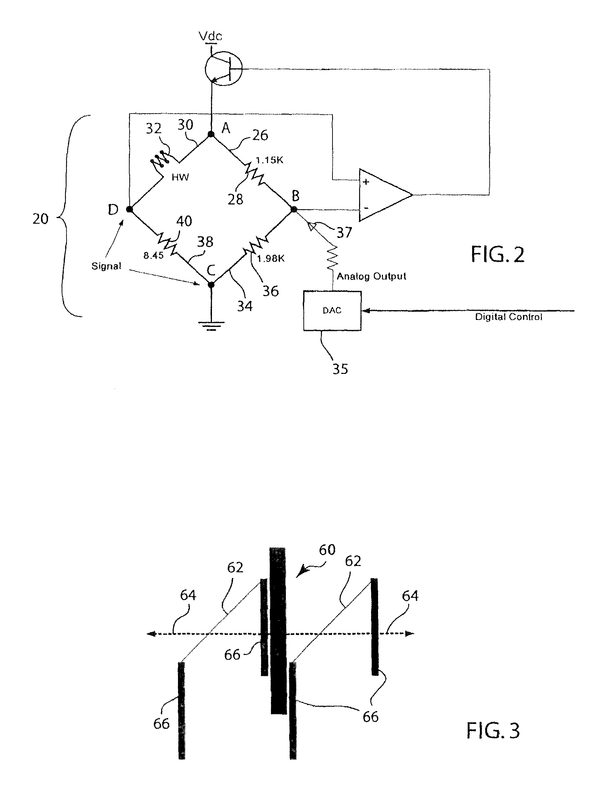

Referring now to Figures 2 and 3, a digital-to-analog converter 35 is integral to each sensor bridge circuit 20 and is used to match the sensing elements 32, 62, removing any variations intrinsic to sensor production. This is normally done when there is a known zero gas flow condition. In the present invention, where a Wheatstone bridge circuit 20 is used in conjunction with each sensing element 32, 62, the DAC 35 injects an output voltage 37 into an arm 26, 66 of the bridge circuit 20. In an iterative process, this output voltage 37 is adjusted until the signal derived from the bridge circuit 20 is equal to, in this application, 200 mV.

Still referring to Figures 2 and 3, conversion of this electrical output voltage 37 to a more meaningful gas flow value is done using a lookup table. This table is created under known and controlled conditions as pertaining to temperature, pressure and gas composition. Any deviation from these conditions will result in inaccuracies when the lookup table is accessed. The zeroing process previously described aligns each sensing element 32, 62 with the table, such that 200 mV is equivalent to zero gas flow. This zeroing process is also beneficial when temperature and gas composition deviate from that which the lookup table was created. Because of the nature of respiratory therapy, temperature and gas composition will change frequently, resulting in the sensor signals drifting from their ideal zero flow value. This will result in measurement inaccuracies.

Referring now to Figure 3 in this application, the sensing elements 62 are not only used to measure gas flow, they are also used to identify flow direction 64. The post element 60 diverts gas flow away from whichever one of the sensing elements 62 is downstream from the flow direction 64, sensing element 62 resulting in a diminished signal. The zeroing procedure described above matches the sensing elements 62 so that a direct comparison of signal values is appropriate for determining the flow direction 64. However, as continuous respiratory therapy can last for weeks at a time and the patient airway is not a controlled environment, the characteristics of the sensing elements 62 are likely to change, possibly resulting in signal divergence. This will result in flow direction 64 misidentification and flow measurement inaccuracies.

Because of the nature of respiratory therapy, it is not uncommon for sensing elements 62 to be replaced periodically for cleaning. This may occur when the patient is connected to the ventilator. A new or cleaned sensing element 62 with DAC values associated with a previous sensing element in another system will likely result in a mismatch between the sensing element 62 and subsequent flow measurement inaccuracies as described above.

In all the cases of measurement inaccuracies of the prior art, a periodic zeroing procedure during therapy would greatly alleviate the inaccuracies. In this application, however, the ventilator control computer cannot create a guaranteed zero flow condition at the wye piece of the system where the sensing elements 62 are positioned, eliminating the possibility of forced zeroing during therapy. This inability to create a zero flow condition is usually due to patient interaction such as spontaneous breathing.

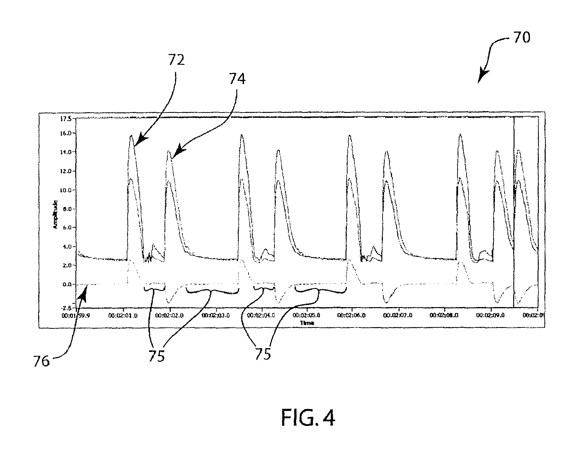

However, at the wye piece, the sensing elements 62 are not subjected to base flow that the ventilator may be delivering to the rest of the system. As a result, a zero gas flow condition will exist during each breath cycle. Referring now to Figure 4, an inspiration/expiration analysis 70 corresponding to the operation of the present invention is depicted. In this inspiration/expiration analysis 70, the inspiration signals 72 represent the voltage over the sensing elements while the patient is inhaling, and the expiration signals 74 indicate the voltage drop of the sensing elements while the patient is exhaling. The net flow 76 in liter/min shows the difference between the inspiration signal 72 and the expiration signal 74. The zero flow condition 75 represents the point between the patient inhaling and exhaling, at which time, there is no air flow.

At zero gas flow 75, the signals derived from the sensors will be at a minimum, providing the opportunity for comparison with their ideal zero flow value, in this case 200 mV. Referring still to Figure 4, the inspiration signal 72 and the expiration signal 74 are shown to converge at the zero flow condition 75. As is stated previously, when the inspiration signal 72 and the expiration signal 74 reach a zero flow condition 75, their value should be equal, and may then be compared to the ideal zero flow rate. It should be noted that the ideal zero flow rate may vary depending upon the particular system and sensing circuit employed, however, in a preferred embodiment a 200 mV ideal zero flow value is used. When the zero flow condition 75 is reached, the values of the inspiration signal 72 and expiration signal 74 will be compared to the ideal zero flow value with an appropriate computer system running software configured to do so. The computer system and software will also be configured to adjust the DAC accordingly to compensate for the differences between the inspiration and expiration signal 72, 74 and the ideal zero flow value.

The sensor circuitry (Figure 3) and associated software that obtains the sensor signals and provides flow and direction data to the main ventilator computer is capable of identifying a zero flow condition, a condition that will occur at least once each breath cycle. By identifying the minimum signal value from each element obtained over a period of time guaranteed to have encompassed at least one breath cycle, these minimum values can be compared to the ideal zero flow value of 200 mV. Adjusting the digital to analog conversion accordingly can compensate for deviations from the ideal. This will reduce errors associated with any of the issues detailed above.

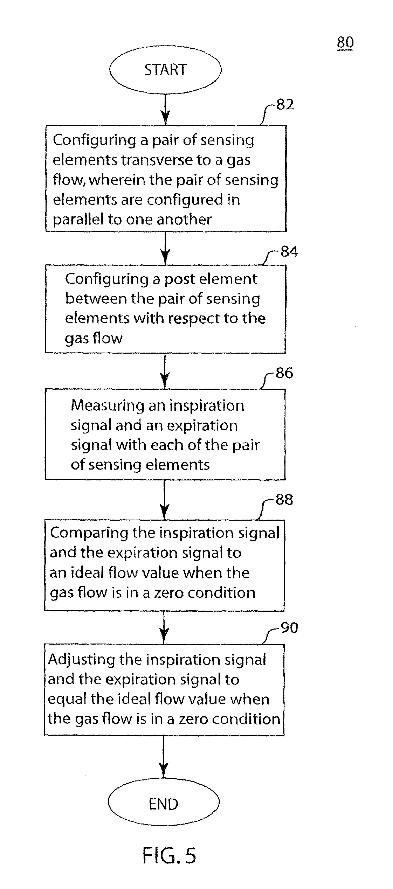

Referring now to Figure 5, a dynamic compensation method 80 of an embodiment of the present invention is depicted in flow chart form. In step 82, a pair of sensing elements are configured transverse to a gas flow, wherein the pair of sensing elements are configured parallel to one another. In step 84, a post element is configured between the pair of sensing elements with respect to the gas flow. In such a configuration, the post element shields one of the pair of sensing elements when the gas flow is in an inspiration direction, and the other sensing element when the gas flow is in an expiration direction. In step 86, an inspiration signal and an expiration signal are measured with each of the pair of sensing elements. In step 88, when the gas flow is in a zero condition between the inspiration direction and the expiration direction, the inspiration signal and the expiration signal are compared to an ideal flow value. In a preferred embodiment, the ideal flow rate equals 200 mV. In step 90, the inspiration signal and the expiration signal are adjusted to equal the ideal flow rate when the gas flow is in a zero condition. It should also be noted that the method may be implemented in a software application, wherein the sensing elements collect the inspiration and expiration signals, and send these values to a processor, wherein the software application, stored in a storage media, compares the inspiration and expiration signals while in a zero condition, to the ideal flow value. The computer application would also be configured to send a signal to the digital to analog converter in order to appropriately adjust the digital analog converters output voltage to the sensing circuit.

Various aspects of the present invention take advantage of three things regarding the application and technology employed; first with respect to the application, the sensor is located at the wye piece of the patient breathing circuit. During respiratory therapy, a base, or bias flow, usually exists to guarantee fresh gasses are available to a patient when either a spontaneous or mechanical breath is delivered. This base flow travels through the breathing circuit but is only seen at the wye during a breath. If this were not the case, there could not be a guarantee of a zero flow condition during each breath cycle.

Second, with respect to the technology, hot wire anemometry is used in a constant temperature configuration. This means that more gas flow results in more current required to keep the hot wire elements at their nominal temperature. This current directly correlates to signal level. Liquid and solid deposits accumulating on the hot wire elements have been shown to also increase the current required to keep the elements at their nominal temperature. Only at zero flow then will the signals derived from the sensors be at a minimum.

Lastly, with respect to the application/technology, the sensor consists of two independent, identical hot wire sensor circuits with a shadowing element placed between them designed to reduce the signal level of the downstream element when gas flow is non-zero. This is critical to having the capability to identify flow direction and balance the circuits through digital to analog conversion as conditions change.

Various aspects of the present invention further provide compensation of the flow sensor such that flow direction identification and measured flow rates are more accurate during respiratory therapy. The dynamic nature of various embodiments of the invention is such that patient therapy does not need to be interrupted during this procedure and no human intervention is required. Prior procedure would require removal of the flow sensor from the breathing circuit, sealing of the sensor to create zero flow, then a button push on the ventilator to notify that a zero flow condition exists before a zeroing procedure could be performed.

The present invention has been described in terms of specific embodiments incorporating details to facilitate the understanding of the principals of construction and operation of the invention. Such reference herein to specific embodiments and details thereof is not intended to limit the scope of the claims appended hereto. It will be apparent to those skilled in the art that modifications may be made in the embodiment chosen for illustration without departing from the spirit and scope of the invention.

## Claims

1. A system for dynamically compensating a bidirectional flow sensor, the system comprising:
a first and second sensing circuit (20), each of the first and second sensing circuits (20) including a sensing element (32, 62), wherein the first and second sensing elements (32, 62) are configured parallel to one another and transverse to a gas flow (64);
a post element (60) configured between the first and second sensing elements (32, 62), wherein the post element (60) is further configured to shield the first sensing element (32, 62) when the gas flow (64) is in an inspiration direction, and is configured to shield the second sensing element (32, 62) when the gas flow (64) is in an expiration direction,
wherein when the gas flow (64) is in a zero condition between the inspiration direction and the expiration direction, an inspiration signal and an expiration signal collected by the first and second sensing elements (32, 62) are compensated to equal an ideal flow value.

2. The system as claimed in claim 1, further comprising a digital to analog converter (DAC) (35) coupled with the first sensing circuit and the second sensing circuit (20).

3. The system as claimed in claim 2, wherein an output voltage (37) of the DAC (35) compensates the inspiration and expiration signals.

4. The system as claimed in any preceding claim, wherein the first and second sensing circuits (20) are bridge circuits.

5. The system as claimed in claim 4, wherein the bridge circuits include a plurality of bridge arms (26, 30, 34, 38), further wherein any of the plurality of bridge arms (26, 30, 34, 38) is the sensing element (32, 62).

6. The system as claimed in any preceding claim, wherein the ideal zero flow value = 200 mV.

7. A method of dynamically compensating a bi-directional sensor, the method comprising:
configuring (82) a pair of sensing elements (32, 62) transverse to a gas flow (64), wherein the pair of sensing elements (32, 62) are configured in parallel to one another;
configuring (84) a post element (60) between the pair of sensing elements (32, 62) with respect to the gas flow;
measuring (86) an inspiration signal and an expiration signal with each of the pair of sensing elements (32, 62);
comparing (88) the inspiration signal and the expiration signal to an ideal flow value when the gas flow is in a zero condition; and
adjusting (90) the inspiration signal and the expiration signal to equal the ideal flow value when the gas flow is in a zero condition.

8. The method as claimed in claim 7, wherein the post element (60) is further configured to shield a first one of the pair of sensing elements (32, 62) when the gas flow is in an inspiration direction, and is configured to shield a second one of the pair of sensing elements (32, 62) when the gas flow is in an expiration direction.

9. An apparatus for dynamically compensating a bi-directional flow sensor, the apparatus comprising:
a pair of sensing elements (32, 62) coupled to a pair of sensing circuits (20), wherein the pair of sensing elements (32, 62) are configured parallel to one another and transverse to a gas flow (64); and
a post element (60) configured between the pair of sensing elements (32, 62),
wherein when the gas flow is in a zero condition, an inspiration signal and an expiration signal collected by the pair of sensing elements (32, 62) are compared to an ideal flow value a digital to analog converter is adjusted to compensate the inspiration signal and expiration signal to equal the ideal flow value.
